# EUROPÄISCHE PATENTSCHRIFT

(11) **EP 4 109 066 B1**
(45) Veröffentlichungstag und Bekanntmachung des Hinweises auf die Patenterteilung: **19.02.2025**
(21) Anmeldenummer: 21181686.3
(22) Anmeldetag: 25.06.2021
(51) Int. Cl.: G01M 3/32, A61M 15/00

(54) **VORRICHTUNG ZUM PRÜFEN DER DICHTIGKEIT EINER PUMPENBAUGRUPPE FÜR HOCHDRUCKSPRÜHAPPLIKATOREN**
DEVICE FOR LEAKAGE TESTING OF A PUMP ASSEMBLY FOR HIGH PRESSURE SPRAY APPLICATORS
DISPOSITIF DE CONTRÔLE DE L'ÉTANCHÉITÉ D'UN ENSEMBLE DE POMPES POUR APPLICATEURS DE PULVÉRISATION À HAUTE PRESSION

(43) Veröffentlichungstag der Anmeldung: 28.12.2022
(73) Patentinhaber: Harro Höfliger Verpackungsmaschinen GmbH, 71573 Allmersbach im Tal (DE)
(72) Erfinder: Wolbers, Kai, 71573 Allmersbach im Tal (DE)
(74) Vertreter: Gleim Petri Patent- und Rechtsanwaltspartnerschaft mbB

(56) Entgegenhaltungen:
- EP-A1- 3 299 144
- US-A1- 2008 202 526
- US-A1- 2013 041 241
- US-A1- 2019 113 418

## Beschreibung

### TECHNISCHES GEBIET

Die Erfindung betrifft eine Vorrichtung zum Prüfen der Dichtigkeit einer Pumpenbaugruppe für Hochdrucksprühapplikatoren. Bei derartigen Hochdrucksprühapplikatoren kann es sich insbesondere um Hochdruckzerstäuber wie Inhalatoren handeln, aber auch um Sprays wie beispielsweise um Nasen- oder Augensprays. Sprühapplikatoren werden vor allem dann verwendet, wenn die Verabreichung eines Wirkstoffs dosisabhängig und/oder abhängig von der Tröpfchengröße ist.

Inhalatoren sind medizinische Geräte (Applikatoren) zur Erzeugung von Aerosolen oder Dämpfen; die Aerosole oder Dämpfe können dann von Patienten eingeatmet werden. Inhalatoren werden insbesondere bei der Behandlung von verschiedenen Atemwegserkrankungen wie beispielsweise von Asthma oder von COPD (Chronic Obstructive Pulmonary Disease, chronisch-obstruktive Lungenkrankheit) eingesetzt. Durch die Inhalatoren können Wirkstoffe in die Lunge gelangen, die dort lokal begrenzt wirken. Dadurch können Nebenwirkungen vermindert werden; auch ist häufig lediglich eine geringere Dosis des Wirkstoffs erforderlich. Der Wirkstoff kann über die Alveolen der Lunge auch in den Blutkreislauf gelangen und dort systemisch wirken. Auch hier sind häufig geringere Dosen ausreichend, da der First-Pass-Effekt der Leber umgangen wird. Darüber hinaus gelangen die Wirkstoffe aufgrund der großen Resorptionsfläche der Lunge schneller in den Blutkreislauf, so dass der Effekt sehr rasch eintritt.

### STAND DER TECHNIK

Die Inhalatoren können beispielsweise als Soft Mist Inhaler (SMI, Hochdruckzerstäuber) ausgebildet sein. Derartige Inhalatoren werden insbesondere in der WO 91/14468 A1, der WO 97/12687 A1 oder der WO 2009/047173 A2 beschrieben. Die Soft Mist Inhaler weisen einen Zerstäuber mit einer mechanischen Pumpe auf, durch die eine langanhaltende feine Sprühwolke eines Fluids erzeugt werden kann. Bei dem Fluid handelt es sich in der Regel um eine wässrige oder ethanolische Lösung des Wirkstoffs. Dabei werden keine Treibmittel für die Erzeugung des Sprühnebels benötigt. Das Fluid liegt in der Regel in einer Kartusche vor. Nach dem Verbrauch einer solchen Kartusche kann diese häufig zumindest einige Male ersetzt werden.

Der Inhalator selbst besitzt eine stabile Kapillare, die in die Kartusche mit dem Fluid eingeführt wird. Die Kapillare taucht ein Stück weit in das Fluid ein, so dass sich dieses durch Kapillarkräfte innerhalb der Kapillare nach oben zieht. Im oberen Bereich der Kapillare sitzt ein Hochdruck-Rückschlagventil zur Dosierung des Fluids. Dieses Rückschlagventil dient dabei sowohl zur Absperrung als auch zur Steuerung des Durchflusses des Fluids innerhalb der Kapillare. Das Fluid kann innerhalb der Kapillare nach oben fließen, jedoch nicht wieder zurück nach unten und damit wieder zurück in die Kartusche.

Als Hochdruck wird in der Regel ein Druck von über 10 bar bezeichnet. Im vorliegenden Fall soll das Hochdruckrückschlagventil einem Druck zwischen 100 bar und 1.000 bar standhalten, bevorzugt einem Druck von etwa 250 bar.

Die Kapillare endet mit ihrem oberen Ende in einer Hochdruck-Pumpenbaugruppe, die die zentrale Einheit der Soft Mist Inhaler - und auch der anderen Sprühapplikatoren - darstellt. Die korrekte Montage dieser Pumpenbaugruppe ist für eine einwandfreie Funktion des Inhalators maßgeblich, so dass bereits geringfügige Fehler oder Abweichungen zu einer Fehlfunktion des Inhalators führen können.

Nach der vollständigen Montage des Inhalators findet in der Regel eine Qualitätsprüfung statt. Durch die Qualitätsprüfung können Fehlfunktionen des Inhalators noch vor der Auslieferung entdeckt werden, so dass keine nichtspezifikationsgerechten Inhalatoren in den Vertrieb gelangen. Darüber hinaus muss auch sichergestellt werden, dass die Dosierung des Fluids reproduzierbar erfolgt. Eine solche Überprüfung der Inhalatoren kann im Rahmen einer Vollprüfung (100%-Prüfung) erfolgen, bei der sämtliche Inhalatoren routinemäßig entsprechend getestet werden. Es wäre auch möglich, die Überprüfung der Inhalatoren im Rahmen von Stichprobenprüfungen vorzunehmen.

Aus der WO 2017/060328 A1 (US 2019/0113418 A1) ist ein Prüfsystem und ein Prüfverfahren bekannt, bei der der fertig montierte Inhalator mit einem Prüf-Fluid getestet wird. Der fertige Inhalator wird dazu an eine Prüf-Kartusche mit dem Prüf-Fluid angeschlossen und mehrere Male maschinell betätigt. Dabei werden verschiedene Messungen, insbesondere hinsichtlich der Verteilung des Sprühnebels und der Menge des zerstäubten Fluids vorgenommen. Sofern der Inhalator bei dieser Prüfung keine korrekten Messwerte liefert, wird der Inhalator aussortiert. Allerdings ist der Ausschuss von Inhalatoren zu diesem Zeitpunkt wirtschaftlich ungünstig, da die Wertschöpfungskette bereits nahezu abgeschlossen ist. Insbesondere muss bei einer Fehlfunktion lediglich eines Bauteils dennoch der gesamte Inhalator ausgeschieden werden.

Darüber hinaus ist in diesem Stadium nach der vollständigen Montage eine Ursachenklärung bei nicht bestandener Prüfung gar nicht oder nur mit einem erheblichen Aufwand - insbesondere einer zumindest teilweisen Demontage des Inhalators - möglich. Eine solche Ursachenklärung ist insbesondere dann wichtig, wenn es bei mehreren Inhalatoren zu ähnlichen Fehlfunktionen kommt. Da unterschiedliche Bauteile gemeinsam getestet und überprüft werden, kann es jedoch zu einer Fehlerverschleppung kommen, so dass eine eindeutige Zuordnung der Fehlfunktion zu einem bestimmten Bauteil nicht mit Sicherheit möglich ist. Bei einer Einzelprüfung auf lediglich eine einzelne Funktion kann dagegen rasch erkannt werden, wo das Problem liegt und welches Bauteil möglicherweise fehlerhaft ist.

Aus der US 2008/0202526 A1, der US 2013/0041241 A1 und der EP 3 299 144 A1 sind verschiedene weitere Anwendungen bekannt, bei denen mittels Unter- oder Überdruck eine Überprüfung auf das Vorhandensein von Leckagen erfolgen soll.

### DARSTELLUNG DER ERFINDUNG

Ausgehend von diesem vorbekannten Stand der Technik liegt der Erfindung die Aufgabe zugrunde eine verbesserte Prüfvorrichtung anzugeben, mittels der eine Einzelprüfung der Dichtigkeit der Pumpenbaugruppe möglich ist.

Die erfindungsgemäße Prüfvorrichtung ist durch die Merkmale des Hauptanspruchs 1 gegeben. Sinnvolle Weiterbildungen der Erfindung sind Gegenstand von sich an diesen Anspruch anschließenden weiteren Ansprüchen.

Die erfindungsgemäße Vorrichtung zum Prüfen der Dichtigkeit einer Pumpenbaugruppe von Inhalatoren weist eine Pumpenbaugruppe mit einer Düseneinheit auf, wobei die Düseneinheit druckdicht an einem Gehäuse gelagert ist. Die Vorrichtung besitzt ein Kopplungselement, durch das die Prüfvorrichtung an der Pumpenbaugruppe angeschlossen werden kann. Darüber hinaus besitzt die Prüfvorrichtung eine Erzeugereinheit für Unterdruck und/oder Überdruck, die an dem Kopplungselement angeschlossen ist, und eine Sensoreinheit, durch die der Druck innerhalb der Pumpenbaugruppe gemessen werden kann.

Durch den Einsatz einer Erzeugereinheit für Unterdruck und/oder Überdruck kann die Dichtigkeit der Pumpenbaugruppe ohne den Einsatz einer Prüf-Flüssigkeit überprüft werden. Dadurch entfällt eine aufwändige Reinigung der Pumpenbaugruppe nach der Qualitätsprüfung, so dass der Montageprozess des Inhalators insgesamt deutlich beschleunigt werden kann. Darüber hinaus kann durch den Wegfall des Reinigungsprozesses eine 100%-Prüfung realisiert werden.

Die erfindungsgemäße Prüfvorrichtung ermöglicht eine Einzelprüfung, durch die die Dichtigkeit der Pumpenbaugruppe direkt in der Prozesslinie überprüft werden kann. Mit der erfindungsgemäßen Prüfvorrichtung ist darüber hinaus eine Überprüfung der Pumpenbaugruppe unmittelbar nach deren Montage möglich. Die Prüfung kann dadurch sehr früh in der Prozesskette und damit auch sehr früh in der Wertschöpfungskette erfolgen, so dass bei der Montage der Inhalatoren Zeit und Geld gespart werden kann.

Vorzugsweise kann die Prüfvorrichtung eine Vakuumpumpe als Erzeugereinheit für Unterdruck aufweisen. Entsprechende Druckanstiegstests haben sich als besonders schnell und als ausreichend genau bezüglich der Auflösung herausgestellt. Dabei wird ein definierter Unterdruck an die Pumpenbaugruppe angelegt und der Druckanstieg als Maß für die Größe der Leckagen gemessen.

Die Pumpenbaugruppe weist eine Düseneinheit auf. Diese Düse stellt eine Leckage der Pumpenbaugruppe dar, die jedoch sehr klein ist. Diese Leckage kann über mehrere Proben ermittelt werden und für die Bestimmung der eigentlichen Leckagen als Offset abgezogen werden. In einer bevorzugten Ausführungsform kann die Prüfvorrichtung ein Verschlusselement aufweisen, durch das die Sprühöffnung der Pumpenbaugruppe und damit die Leckage durch die Düseneinheit abgedichtet werden kann. Bei dem Verschlusselement kann es sich beispielsweise auch um eine Flüssigkeit handeln, die in die Sprühöffnung der Pumpenbaugruppe eingebracht wird. Die Flüssigkeit sollte in diesem Fall rasch und rückstandslos verdunsten, wie dies beispielsweise bei niederkettigen Alkoholen, insbesondere bei Ethanol, der Fall ist.

Die Düseneinheit ist druckdicht an einem Gehäuse gelagert. Die druckdichte Lagerung kann insbesondere mittels zumindest einer Dichtung erfolgen. Die Dichtigkeit der Pumpenbaugruppe hängt dabei maßgeblich von der Funktionsfähigkeit dieser zumindest einen Dichtung zwischen der Düseneinheit und dem Gehäuse ab.

In einer ersten Ausführungsform der Erfindung kann das Kopplungselement eine Kapillare als Adapter aufweisen, die druckdicht in die Düseneinheit der Pumpenbaugruppe eingesteckt werden kann. Da die Düseneinheit im späteren Verlauf der Montage mit einer entsprechenden Kapillare bestückt wird, ist die Düseneinheit bereits entsprechend ausgestaltet, so dass auf diese Weise ein einfacher Anschluss an die Prüfvorrichtung ermöglicht wird.

In einer zweiten Ausführungsform der Erfindung kann die Pumpenbaugruppe bereits mit einer Kapillare ausgestattet sein. In diesem Fall kann das Kopplungselement über ein Ansatzstück lösbar an dem freien Ende der Kapillare befestigt werden.

Alternativ dazu könnte die Erzeugereinheit für Unterdruck und/oder Überdruck auch mittels eines Kopplungselements an der Düseneinheit der Pumpenbaugruppe angeschlossen werden. In diesem Fall müsste dann die an der Pumpenbaugruppe angeschlossene Kapillare oder das entsprechende Ansatzstück für die Kapillare an der Pumpenbaugruppe abgedichtet werden.

Die Bedienung der Prüfvorrichtung kann manuell, semiautomatisch oder vollautomatisch umgesetzt werden. Die Prüfvorrichtung kann in den Montageprozess des Inhalators so integriert werden, dass jede Pumpenbaugruppe von der Prüfvorrichtung getestet wird, so dass eine 100%-Prüfung erfolgt. Es wäre jedoch auch eine Stichprobenprüfung möglich.

Eine entsprechende Prüfung würde mit folgenden Verfahrensschritten ablaufen:
1. Die Prüfvorrichtung wird druckdicht an der Pumpenbaugruppe angeschlossen.
2. In der Druckkammer (Innenraum der Düseneinheit und der Kapillare der Prüfvorrichtung oder der Pumpenbaugruppe) wird ein Unterdruck oder ein Überdruck erzeugt.
3. Der Druckanstieg beziehungsweise der Druckabfall innerhalb der Druckkammer wird mittels einer Sensoreinheit überwacht.

Die Messwerte der Sensoreinheit können anschließend elektronisch ausgelesen werden, wodurch der Druckanstieg beziehungsweise der Druckabfall ausgewertet werden kann. Auf der Basis dieser Auswertung kann anschließend eine manuelle oder automatische Bewertung der Baugruppenfunktion erfolgen.

Grundsätzlich könnten auch mehrere Prüfvorrichtungen in den Montageprozess des Inhalators integriert werden. So könnte beispielsweise zunächst eine erste Ausführungsform der Prüfvorrichtung vorgesehen werden, bei der die Pumpenbaugruppe ohne Kapillare getestet würde. Im weiteren Verlauf des Montageprozesses könnte dann eine zweite Ausführungsform der Prüfvorrichtung vorgesehen werden, um die Pumpenbaugruppe mit eingesetzter Kapillare erneut zu testen.

Weitere Vorteile und Merkmale der Erfindung sind den in den Ansprüchen ferner angegebenen Merkmalen sowie dem nachstehenden Ausführungsbeispiel zu entnehmen.

### KURZE BESCHREIBUNG DER ZEICHNUNG

Die Erfindung wird im Folgenden anhand des in der Zeichnung dargestellten Ausführungsbeispiels näher beschrieben und erläutert. Es zeigt:
- Fig. 1: einen schematischen Längsschnitt durch eine erste Ausführungsform der erfindungsgemäßen Prüfvorrichtung, die an der Kapillare einer Pumpenbaugruppe angeschlossen ist,
- Fig. 2: einen schematischen Längsschnitt durch eine zweite Ausführungsform der erfindungsgemäßen Prüfvorrichtung die an der Düseneinheit einer Pumpenbaugruppe angeschlossen ist.

### WEGE ZUM AUSFÜHREN DER ERFINDUNG

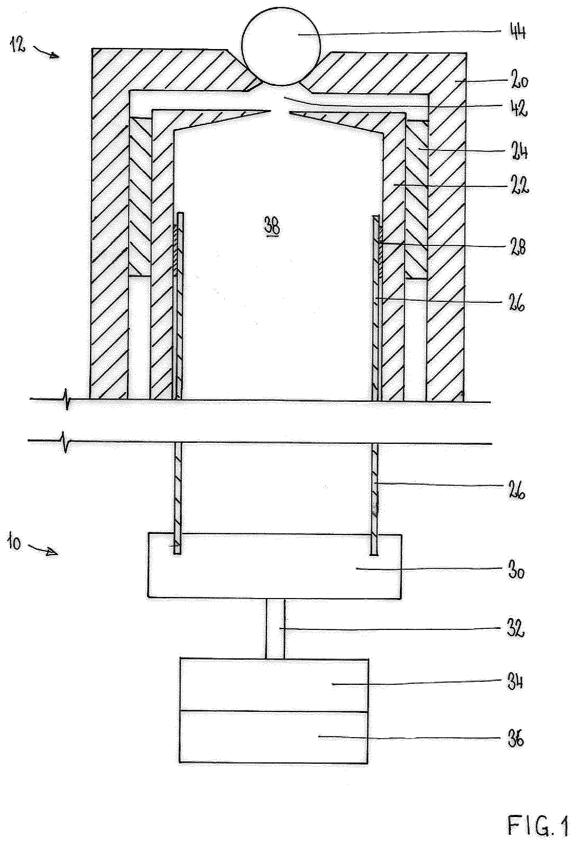

Eine erste Ausführungsform der erfindungsgemäßen Vorrichtung 10 zum Prüfen der Dichtigkeit einer Pumpenbaugruppe 12 für Inhalatoren ist in Fig. 1 schematisch dargestellt.

Die Pumpenbaugruppe 12 besitzt ein Gehäuse 20 sowie eine Düseneinheit 22. Zwischen der Düseneinheit 22 und dem Gehäuse 20 befindet sich eine Dichtung 24, so dass die Düseneinheit 22 druckdicht an dem Gehäuse 20 gelagert ist. Die Düseneinheit 22 ist an dem oberen Ende einer Kapillare 26 befestigt. Zwischen der Kapillare 26 und der Düseneinheit 22 befindet sich eine weitere Dichtung 28, die zu der Düseneinheit 22 gehört.

Insbesondere die Dichtigkeit der Dichtung 24 zwischen dem Gehäuse 20 und der Düseneinheit 22 und die Dichtigkeit der Dichtung 28 zwischen der Düseneinheit 22 und der Kapillare 26 soll mit der erfindungsgemäßen Prüfvorrichtung 10 überprüft werden.

Im vorliegenden Beispielsfall ist die Kapillare 26 Bestandteil eines Kopplungselements 30. Mittels des Kopplungselements 30 kann die Prüfvorrichtung 10 an der Pumpenbaugruppe 12 angeschlossen werden. Das Kopplungselement 30 ist über eine Leitung 32 mit einer Vakuumpumpe 34 sowie mit einer Sensoreinheit 36 verbunden. Über die Vakuumpumpe 34 kann im Innenraum der Kapillare 26 und der Düseneinheit 22 (Druckkammer 38) ein definierter Unterdruck erzeugt werden.

Die Sensoreinheit 36 kann messen und aufzeichnen, wie sich der Unterdruck der Druckkammer 38 über einen definierten Zeitraum ändert. Sofern die Dichtungen 24, 28 einwandfrei funktionieren, sollte es daher nach einer gewissen Beruhigungszeit zu keinem nennenswerten Druckanstieg in der Druckkammer 38 kommen.

Ein geringfügiger Druckanstieg in der Druckkammer 28 kann daraus resultieren, dass durch die Sprühöffnung 42 in dem Gehäuse 20 der Pumpenbaugruppe 12 eine gewisse Mini-Leckage besteht. Die Leckage der Sprühöffnung 42 kann durch mehrere Kallibrierungsmessungen genau ermittelt werden. Bei der eigentlichen Prüfung kann diese Undichtigkeit dann von den ermittelten Messwerten abgezogen werden.

Bei der vorliegenden Ausführungsform weist die Prüfvorrichtung 10 ein Verschlusselement 44 auf, durch das die Sprühöffnung 42 der Pumpenbaugruppe 12 abgedichtet werden kann. Auf diese Weise müssen keine zusätzlichen Kallibrierungsmessungen durchgeführt werden, da keine Leckage durch die Sprühöffnung 42 mehr besteht. Daher sollte es bei der Prüfung auch zu keinem Druckanstieg in der Druckkammer 38 kommen. Bei dem Verschlusselement 44 kann es sich beispielsweise auch um eine Flüssigkeit handeln, die in die Sprühöffnung 42 eingebracht wird. Diese Flüssigkeit sollte rasch und rückstandslos verdunsten; dies ist insbesondere bei niederkettigen Alkoholen wie beispielsweise bei Ethanol der Fall.

Im Gegensatz zu der in der Zeichnung dargestellten Ausführungsform könnte statt der Vakuumpumpe 34 auch eine Erzeugereinheit für Überdruck eingesetzt werden. In diesem Fall würde in der Druckkammer 38 ein Überdruck erzeugt werden. Die Sensoreinheit 36 würde in diesem Fall messen, ob der Überdruck in der Druckkammer 38 über einen definierten Zeitraum abfällt.

Im Gegensatz zu dem in der Zeichnung dargestellten Ausführungsbeispiel könnte die Pumpenbaugruppe 12 bei der Dichtigkeitsprüfung bereits mit einer Kapillare 26 ausgestattet sein. In diesem Fall würde das Kopplungselement ein entsprechendes Ansatzstück aufweisen, das lösbar an dem freien Ende der Kapillare befestigt werden kann.

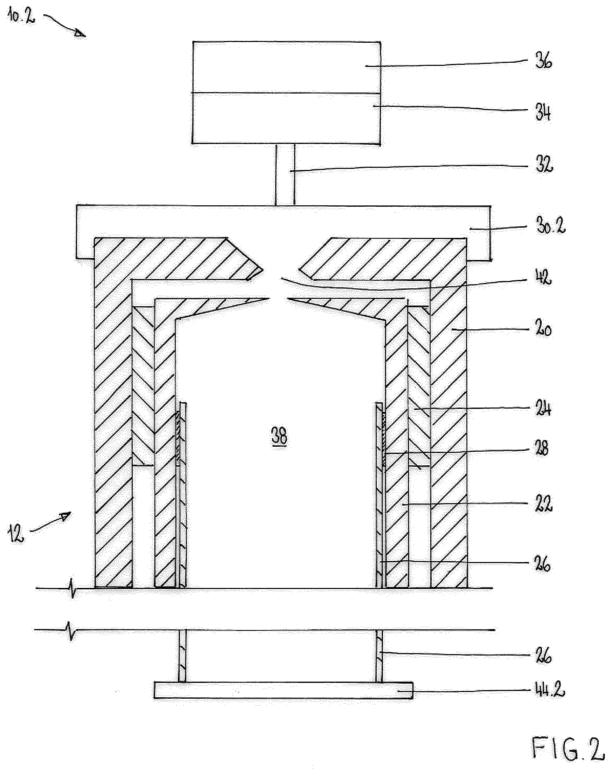

Eine zweite Ausführungsform der erfindungsgemäßen Vorrichtung 10.2 zum Prüfen der Dichtigkeit einer Pumpenbaugruppe 12 für Inhalatoren ist in Fig. 2 schematisch dargestellt. Mittels eines Kopplungselements 30.2 kann die Prüfvorrichtung 10.2 an der Düseneinheit 22 der Pumpenbaugruppe 12 angeschlossen werden. Das Kopplungselement 30.2 ist über eine Leitung 32 mit der Vakuumpumpe 34 sowie mit der Sensoreinheit 36 verbunden. Über die Vakuumpumpe 34 kann im Innenraum der Düseneinheit 22 (Druckkammer 38) ein definierter Unterdruck erzeugt werden. Die an der Pumpenbaugruppe 12 vorhandene Kapillare 26 wird dazu über ein Verschlusselement 44.2 abgedichtet.

Sofern bei dieser Ausführungsform keine Kapillare 26 an der Pumpenbaugruppe 12 vorhanden ist, kann die Pumpenbaugruppe 12 auch über ein stiftförmiges Verschlusselement abgedichtet werden.

## Patentansprüche

1. Vorrichtung (10, 10.2) zum Prüfen der Dichtigkeit einer Pumpenbaugruppe (12) für Sprühapplikatoren,
wobei die Pumpenbaugruppe (12) eine Düseneinheit (22) aufweist, die druckdicht an einem Gehäuse (20) gelagert ist, **dadurch gekennzeichnet, dass** die Vorrichtung umfasst:
- ein Kopplungselement (30, 30.2), durch das die Prüfvorrichtung (10, 10.2) an die Pumpenbaugruppe (12) anschließbar ist,
- eine Erzeugereinheit (34) für Unterdruck und/oder Überdruck, die an dem Kopplungselement (30, 30.2) angeschlossen ist,
- eine Sensoreinheit (36), durch die der Druck innerhalb der Pumpenbaugruppe (12) messbar ist.

2. Vorrichtung nach Anspruch 1,
- **dadurch gekennzeichnet, dass**
- die Düseneinheit (22) mittels zumindest einer Dichtung (24) druckdicht an dem Gehäuse (20) gelagert ist.

3. Vorrichtung nach Anspruch 1 oder 2,
- **dadurch gekennzeichnet, dass**
- das Kopplungselement (30) eine Kapillare (26) aufweist,
- die Kapillare (26) in die Düseneinheit (22) der Pumpenbaugruppe (12) druckdicht einsteckbar ist.

4. Vorrichtung nach Anspruch 1 oder 2,
- **dadurch gekennzeichnet, dass**
- die Pumpenbaugruppe eine Kapillare aufweist, die druckdicht an der Düseneinheit befestigt ist,
- das Kopplungselement ein Ansatzstück (40) aufweist, das lösbar an dem freien Ende der Kapillare (26) befestigbar ist.

5. Vorrichtung nach Anspruch 3 oder 4,
- **dadurch gekennzeichnet, dass**
- die Kapillare der Pumpenbaugruppe (12) oder die Kapillare (26) der Prüfvorrichtung (10) mittels zumindest einer Dichtung (28) an der Düseneinheit (22) befestigt oder befestigbar ist.

6. Vorrichtung nach einem der vorstehenden Ansprüche,
- **dadurch gekennzeichnet, dass**
- ein Verschlusselement (48) vorhanden ist, durch das die Sprühöffnung (32) der Pumpenbaugruppe (12) abdichtbar ist.

7. Verfahren zum Prüfen der Dichtigkeit einer Pumpenbaugruppe (12) für Sprühapplikatoren, **gekennzeichnet durch** die folgenden Verfahrensschritte:
- eine Prüfvorrichtung (10, 10.2) wird druckdicht an der Pumpenbaugruppe (12) angeschlossen,
- in einer Druckkammer (38) der Pumpenbaugruppe (12) wird mittels einer Erzeugereinheit (34) für Unterdruck und/oder Überdruck ein Überdruck oder ein Unterdruck erzeugt,
- der Druckanstieg beziehungsweise der Druckabfall innerhalb der Druckkammer (38) wird mittels einer Sensoreinheit (36) überwacht.

8. Verfahren nach Anspruch 7,
- **dadurch gekennzeichnet, dass**
- die Messwerte der Sensoreinheit (36) elektronisch ausgelesen werden, wodurch der Druckanstieg beziehungsweise der Druckabfall ausgewertet werden kann.

9. Verfahren nach Anspruch 8,
- **dadurch gekennzeichnet, dass**
- auf Basis der Auswertung eine manuelle oder automatische Bewertung der Baugruppenfunktion erfolgt.

## Claims

1. Device (10, 10.2) for testing the tightness of a pump assembly (12) for spray applicators,
wherein the pump assembly (12) has a nozzle unit (22), which is mounted in a pressure-tight manner on a housing (20), **characterized in that** the device comprises:
- a coupling element (30, 30.2) by way of which the testing device (10, 10.2) is connectable to the pump assembly (12),
- a generating unit (34) for negative and/or positive pressure, which is connected to the coupling element (30, 30.2),
- a sensor unit (36) by way of which the pressure inside the pump assembly (12) is able to be measured.

2. Device according to Claim 1,
- **characterized in that**
- the nozzle unit (22) is mounted in a pressure-tight manner on the housing (20) by means of at least one seal (24).

3. Device according to Claim 1 or 2,
- **characterized in that**
- the coupling element (30) has a capillary (26),
- the capillary (26) is insertable in a pressure-tight manner into the nozzle unit (22) of the pump assembly (12).

4. Device according to Claim 1 or 2,
- **characterized in that**
- the pump assembly has a capillary which is fastened in a pressure-tight manner to the nozzle unit,
- the coupling element has an attachment (40) which is able to be releasably fastened to the free end of the capillary (26).

5. Device according to Claim 3 or 4,
- **characterized in that**
- the capillary of the pump assembly (12), or the capillary (26) of the testing device (10), is fastened, or able to be fastened, to the nozzle unit (22) by means of at least one seal (28).

6. Device according to one of the preceding claims,
- **characterized in that**
- there is a closure element (48) by way of which the spray opening (32) of the pump assembly (12) is able to be sealed.

7. Method for testing the tightness of a pump assembly (12) for spray applicators, **characterized by** the following method steps:
- a testing device (10, 10.2) is connected in a pressure-tight manner to the pump assembly (12),
- in a pressurized chamber (38) of the pump assembly (12), a positive or a negative pressure is generated by means of a generating unit (34) for negative pressure and/or positive pressure,
- the pressure increase or the pressure drop within the pressurized chamber (38) is monitored by means of a sensor unit (36).

8. Method according to Claim 7,
- **characterized in that**
- the measured values of the sensor unit (36) are read electronically, as a result of which the pressure increase or the pressure drop can be evaluated.

9. Method according to Claim 8,
- **characterized in that**
- a manual or automatic assessment of the assembly function is carried out on the basis of the evaluation.

## Revendications

1. Dispositif (10, 10.2) pour contrôler l'étanchéité d'un module de pompe (12) pour des applicateurs à pulvérisation,
le module de pompe (12) comprenant une unité de buse (22), qui est montée de manière étanche à la pression sur un boîtier (20), **caractérisé en ce que** le dispositif comprend :
- un élément de couplage (30, 30.2), par lequel le dispositif de contrôle (10, 10.2) peut être raccordé au module de pompe (12),
- une unité génératrice (34) de dépression et/ou de surpression, qui est raccordée à l'élément de couplage (30, 30.2),
- une unité de détection (36), par laquelle la pression à l'intérieur du module de pompe (12) peut être mesurée.

2. Dispositif selon la revendication 1,
- **caractérisé en ce que**
- l'unité de buse (22) est montée de manière étanche à la pression sur le boîtier (20) au moyen d'au moins une garniture d'étanchéité (24).

3. Dispositif selon la revendication 1 ou 2,
- **caractérisé en ce que**
- l'élément de couplage (30) possède un capillaire (26),
- le capillaire (26) peut être inséré de manière étanche à la pression dans l'unité de buse (22) du module de pompe (12).

4. Dispositif selon la revendication 1 ou 2,
- **caractérisé en ce que**
- le module de pompe possède un capillaire qui est fixé de manière étanche à la pression à l'unité de buse,
- l'élément de couplage possède un embout (40) qui peut être fixé de manière amovible à l'extrémité libre du capillaire (26).

5. Dispositif selon la revendication 3 ou 4,
- **caractérisé en ce que**
- le capillaire du module de pompe (12) ou le capillaire (26) du dispositif de contrôle (10) est fixé ou peut être fixé à l'unité de buse (22) au moyen d'au moins une garniture d'étanchéité (28).

6. Dispositif selon l'une des revendications précédentes,
- **caractérisé en ce que**
- un élément de fermeture (48) est présent, par lequel l'ouverture de pulvérisation (32) du module de pompe (12) peut être rendue étanche.

7. Procédé pour contrôler l'étanchéité d'un module de pompe (12) pour des applicateurs à pulvérisation, **caractérisé par** les étapes suivantes :
- un dispositif de contrôle (10, 10.2) est raccordé de manière étanche à la pression au module de pompe (12),
- une surpression ou une dépression est générée dans une chambre de pression (38) du module de pompe (12) au moyen d'une unité de génération de dépression et/ou de surpression (34),
- l'augmentation de pression ou la chute de pression à l'intérieur de la chambre de pression (38) est surveillée au moyen d'une unité de détection (36).

8. Procédé selon la revendication 7,
- **caractérisé en ce que**
- les valeurs de mesure de l'unité de détection (36) sont lues électroniquement, ce qui permet d'évaluer l'augmentation de la pression ou la chute de pression.

9. Procédé selon la revendication 8,
- **caractérisé en ce que**
- une évaluation manuelle ou automatique du fonctionnement du sous-ensemble est effectuée sur la base de l'évaluation.
